# EUROPEAN PATENT SPECIFICATION

(11) **EP 2 364 712 B1**
(45) Date of publication and mention of the grant of the patent: **06.03.2013**
(21) Application number: 10156192.6
(22) Date of filing: 11.03.2010
(51) Int. Cl.: A61K 35/74, A61K 31/01, A61K 31/695, A61P 31/02

(54) **Topical cosmetic or pharmaceutical composition comprising probiotic lactobacillus strains and use of same**
Topische kosmetische oder pharmazeutische Zusammensetzung mit probiotischen Lactobacillus-Stämmen und Verwendung davon
Composition cosmétique ou pharmaceutique topique comprenant des souches de lactobacillus probiotiques et son utilisation

(43) Date of publication of application: 14.09.2011
(73) Proprietor: KLOARYS INVESTMENT LTD, Road Town, Tortola (VG)
(72) Inventor: Lopes, Alexandra, 78780, Maurecourt (FR); Lheritier, Anne Marie, 95000, Cergy (FR); Mielcarek, Christine, 60590, Trie Chateau (FR); Malard, Patrice, 31270, Cugnaux (FR)
(74) Representative: Vuille, Roman

(56) References cited:
- EP-A1- 1 110 555
- WO-A1-2004/060416
- US-A- 1 758 937
- US-A1- 2004 022 775
- US-A1- 2008 107 699

## Description

### Field of the Invention

The invention refers to the application of probiotic microorgansims onto healthy skin or mucosa and to subjects affected by skin or mucosal troubles caused by pathogens as well, in particular for balancing or restoring skin or mucosal indigenous microflora in humans.

### Background of the Invention

Probiotics, which have been defined as "live microorgansims which when administrated in adequate amounts confer a beneficial health effect on the host" by the WHO (2001) are becoming more and more popular in the prevention or the treatment of numerous diseases or disorders. This is specially the case for gastro-intestinal troubles such as e.g. antibiotics associated diarrhoea, rotavirus or infectious diarrhoea, etc....

The interest for probiotics is related to an increased understanding on the functions of the indigenous bacterial microflora and the disturbance arising from an imbalanced indigenous microflora. This is particularly well understood concerning the gut microflora and also, to some extent, for the vaginal, oral or skin human microflora. Although most of the resident micro-flora on healthy skin and mucosa can be regarded as harmless or even beneficial, in some cases microbial imbalance is giving rise to diseases like acne, atopy, bacterial vaginosis and others.

Several experimental and clinical studies have assessed already the potential of certain lactobacilli in the prevention or treatment of certain genito-urinary tract infections such as bacterial vulvovaginitis, vaginosis or urinary tract infections and relevant therapy has been proposed for many decades already.

Urogenital infections, either severe or benign, remain a common problem in the female population. Bacterial adherence to the urogenital epithelium is recognized as an important mechanism in the initiation and pathogenesis of urinary tract infections (UTI) and lower vaginal tract infections as well. The urogenital pathogens originate predominantly in the intestinal tract and initially colonize the per-urethral region and ascend into the bladder, resulting in symptomatic or asymptomatic bacterial uria.

Alternatively, these bacteria can invade and then colonize the vagina causing there various types of symptomatic as well as asymptomatic vaginal infections.

The use of bacteria of the autochthonous flora, such as lactobacilli, to exclude urogenital pathogens from colonizing the urogenital tract is an established concept studied rather extensively since years (see e.g. Cadieux et al. - Lactobacillus strains and vaginal ecology - Jama. 287:1940- 41 / 2002)

The main goal of therapy with bio therapeutic agents should be to prevent overgrowth of pathogens until such a time that the normal micro flora can be re-established. In addition, bacterial therapy is considered as "natural" and without side effects in contrast with conventional pharmaceutical treatments.

The proposed therapeutic or prophylactic treatments are performed by administering either orally or locally a dedicated preparation comprising, in combination with a suitable excipient, support or carrier an effective amount of the selected lactobacillus strains. Local administration is usually performed by means of vaginal gellules, capsules or suppositories. In the case of benign infections, e.g. UTI, however such an administration is relatively heavy and frequently perceived as unpleasant.

When proposed for an oral administration said compositions can be provided as food supplements, preferably in the form of ingestible capsules comprising lyophilized microorganisms, or in the form of edible milk based powders, suspensions or emulsions or in the form of edible fermented milk products, e.g. yogurts.

The efficiency of such an administration, however, remains questionable as it has to rely on the adequate survival of the probiotic bacteria through out the all GI track and then to the adequate colonization of the vagina from the transfer of the bacteria from the rectum. The level of acceptance of that type of treatment or prevention method is consequently fairly low.

Obviously, the concerned population is still looking today for an easier and more acceptable, more convenient method of local administration of the active agent, especially for long term treatment.

WO 2001/45721 discloses cosmetic or pharmaceutical compositions containing a bacterial agent, either a bacterial extract or inactivated bacteria of very specific species, useful for stabilizing or regulating the skin ecosystem of mammals. These are, more concretely, L. Johnsonii CNCM 1-1225, Micrococus varians CNCM 1-1568 & 1587 and Bifidobacterium lactis ATCC 27536, which exhibit anti-adhesion properties in relation to the pathogenic flora of the skin.

EP 1 593 382 discloses the use of probiotic lactic bacteria or of culture supernatant there from for balancing the skin immune system, especially when the latter is under stress condition such as an exposure to UV radiation.

EP 1 642 570 discloses cosmetic or pharmaceutical compositions useful for treating sensitive or dry skins in mammals which comprise L. paracasei CNCM 1-2116, fractions of same or related metabolites associated to a strain of species Bifidobacterium longum.

Despite of the interest to apply probiotics directly onto the affected portion of skin or mucosa as suggested e.g. here above attempts to develop commercial products have been hampered due to a lack of stability of the probiotics bacteria strains in cosmetic compositions. Main reason for this is, on first hand, that most cosmetic preparations do contain preservative agents to avoid exogenous bacterial contamination and, on the second hand, that most in not all cosmetic preparations do contain water which is likely to alter the stability and the shelf life of the probiotic bacteria strains.

The purpose of this invention is to provide a new and innovative vehicle suitable for topical administration on a long term basis which overcomes all the drawbacks met previously and which allows simultaneously an improved and faster treatment or restoration of the initial health status of the subject.

### Summary of the Invention

The object of this invention is a device for the extemporaneous preparation of a cosmetic or pharmaceutical composition for topical application consisting of a primary oily phase and a complementary aqueous phase wherein the primary oily phase comprises a suspension of an effective amount of at least one viable probiotic bacteria strain in a non polar mineral or silicone oil, said device consisting of a container comprising two separate compartments each having its own outlet acting as dispensing or dosing or deposition system, wherein the first compartment comprises an oily primary phase as defined here above and wherein the second compartment comprises the complementary aqueous phase as defined here above.

### Detailed description of the Invention

According to the invention the primary phase consists of a non polar oily composition of matter. These conditions are necessary for guaranteeing appropriate survival, especially long term survival during an extended storage period of the probiotic bacteria strains suspended therein.

According to the invention the said primary oily phase selected from a wide range of substances of non polar mineral (or synthetic) and silicone origin, preferably among the cosmetic grade fluids like oils or waxes presenting the adequate viscosity. As examples of mineral oils or waxes one can cite e.g. isohexadecane, isododecane, paraffin oils, liquid Vaseline® or synthetic waxes. As silicone based oils or waxes on can cite e.g. dimethicone, cyclopentasiloxane, cyclohexysiloxane, cyclotetrasiloxane as well as mixtures of same as currently commercialised as cosmetic or pharmaceutical carriers or excipient. This enumeration is, of course, not exhaustive.

An appropriate selection of the components, either as the pure oil or as a more or less complex mixture, allows long term storage of the lactobacillus, usually in the form of lyophilized lactobacillus strains, in the suspension so achieved: long term means at least 6 months or even more, i.e. periods where one notes a decrease of approximately one log CFU per unit during the initial phase of storage and then a quite stabilized evolution of the CFU counts and subsequent 100 % revival of the samples.

Long term viability is further guaranteed by the fact that the said primary oily phase does not comprise any exogenous preservative agent which would interfere negatively with the strains of the suspension.

Also crucial for long term stability and subsequent revival of the probiotic strains is the intrinsic viscosity of the primary oily phase; this would avoid undesired deposition of the strains during storage, consequently preserve adequate dispersion of the strains in the oil media during storage. The desired viscosity of the said primary oily phase can be due to the nature of the selected material, either as the pure oil or as a more or less complex mixture; it can also be achieved by means of conventional viscosifying or stabilizing agents, especially cosmetic grade viscosifying agents.

According to the invention the primary oily phase may comprise from 10⁷ to 10¹¹,preferably from 10⁸ to 10¹⁰ colony forming units (CFU) per g of the selected probiotic lactobacillus strain(s). This would enable to obtain sufficient deposition of viable strains onto the selected skin or mucosa surface in order to achieve the desired effect on site, usually the colonisation of the selected skin or mucosa area where the probiotic lactobacillus can exert their effect in optimal conditions. An amount of ca 10⁵ to 10⁹, preferably from 10⁶ to 10⁸ CFU per surface units (cm²) has been proved as efficient in most of the situations met.

The said probiotic lactobacillus strains are usually present in the suspension in their lyophilized and /or encapsulated form.

The cosmetic composition used in the device of this invention further comprises a so called complementary phase, in fact an aqueous phase the goal of which consists to provide to the end composition all the ingredients or components necessary to obtain the desired cosmetic composition.

Such a complementary aqueous phase can be a solution or even an emulsion comprising appropriate amounts of e.g. emulsifiers, jellifying agents, stabilizers, antioxidants, foaming agents or preservatives, the latter list being not limitative.

The said complementary aqueous phase can further comprise compounds or substances acting as bacterial growth factors and which will exert their specific effect only once the lyophilized lactobacillus strains are exposed to water. Conventional bacterial growth factors are prebiotic fibers or e.g. dedicated natural growth factors like skimmed milk powder (MSK). As long as the probiotic lactobacillus strains remain "encapsulated" or "protected" in the oily phase of the invention from any contact with water their survival and revival capacity is kept intact.

Both the primary anhydrous oily phase and the aqueous complementary phase can be stored separately as long as necessary and the mixed to afford the desired cosmetic composition, usually in the form of an oil-in-water or water-in-oil or even as a more complex emulsion.

According to the invention both the primary anhydrous oily phase and the aqueous complementary phase are stored separately and the end cosmetic composition is prepared extemporaneously, i.e. on site right before using.

It is only once the cosmetic composition is applied onto the selected skin or mucosa area, usually after soft spreading or rubbing of the selected area, that both the primary and the complementary phase are "destructered" and that the strains in suspension in the oily phase come into tight contact with water. Water, which is necessary for the reactivation and subsequent growth of the bacteria, is obviously provided by the cosmetic composition itself but also, depending on the selected area, from the local body fluids; this especially the case of a topical application on the skin or mucosa surface met in the lower urinary or lower vaginal tract, like e.g. vulva.

The probiotic lactobacillus strains used within the frame of the invention have been selected for their capacity to colonize epithelial tissues, e.g. those of the lower urogenital tract, and then to compete with, inhibit or exclude pathogens adhesion from that specific location.

Gram-negative or Gram-positive pathogens such as those mentioned here after are representative of those which are significantly affected by the selected probiotic lactobacillus strains in terms on adhesion, growth or pathogenic activity: Salmonella species, like S. enterica serovar Typhimurium, E. coli, Streptococcus species, e.g. S. agalactiae, Staphylococcus species like S. aureus, Gardnerella species, e.g. G. vaginalis, Prevotella species, e.g. P. bivia; this enumeration is of course not exhaustive.

According to the invention the primary oily phase referred to here above usually comprise at least one strain selected from the species like e.g. : L. plantarum, L.jensenii, L. crispatus, L. gasseri, L. helveticus, L. fermentum, L. rhamnosus, L. casei, L. reuteri or L. acidophilus.

As particularly preferred species, on can further cite the following strains: Lactobacillus plantarum (Heal 9) DSM 15312, Lactobacillus plantarum (Heal 19) DSM 15313, Lactobacillus plantarum (299v) DSM 9843, L. plantarum LP(1) KCTC 3928, Lactobacillus jensenii CNCM I-3217, Lactobacillus gasseri CNCM 1-3218, Lactobacillus jensenii CNCM 1-3219 and Lactobacillus helveticus CNCM 1-3360.

Most of these strains are representative of the healthy human vaginal microflora and have shown their efficacy both in vitro and in vivo and their compatibility with the local microflora.

These prior known strains have been duly registered at the Pasteur Institute, Paris (France), at the Deutsche Sammlung von Mikroorganismen (DE), the Korean Collection for Type Cultures (KCTC) or any equivalent authorized institution following the rules of the Budapest Treaty.

Also, the probiotic lactobacillus strains referred to here above can be used in a quite efficient way for maintaining or restoring a healthy urogenital flora in females, in particular after severe medical treatments like those performed with antibiotics.

Consequently the invention is particularly convenient for preventing or treating urinary tract infections (UTI) and lower vaginal tract infections in females or balancing or restoring the local urinary tract or lower vaginal tract bacterial flora in females.

Topical application is performed by deposition, on the selected area, of an amount providing ca 10⁶ CFU by means of the cosmetic composition per cm2 and subsequent soft spreading or massage.

Topical application can be performed by means of any dispenser as those met in the cosmetic industry for the administration of e.g. viscous liquids, creams, waxes or sticks.

For extemporaneous application one can use a device which consists of a container comprising two separate compartments each having its own outlet acting as dispensing or dosing or deposition system, wherein the first compartment comprises the oily primary phase as defined here above and wherein the second compartment comprises the complementary aqueous phase as defined here above.

### Figures

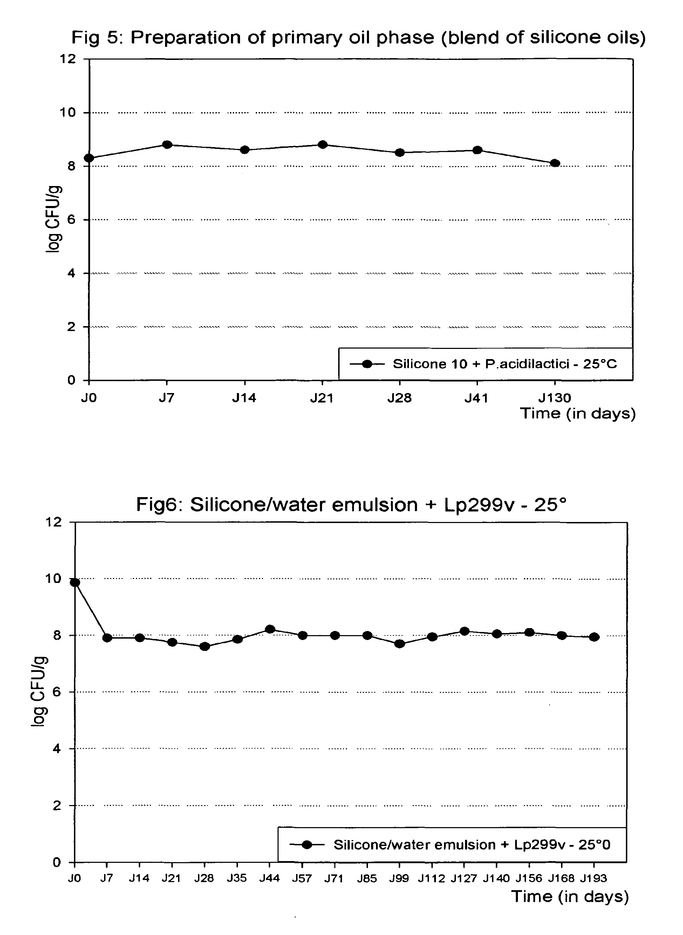

Fig .1 to Fig. 6 illustrate experimental results achieved using selected probiotic lactobacillus under various conditions such as e.g. the nature of the suspension media, the storage period and the storage temperature. These figures are illustrative of the examples listed here below.

### Example 1: Preparation of a primary oil phase (silicone oil)

A portion of 0.60 g of L. plantarum 299v (DSM 9843) or L. plantarum Heal 9 (DSM 15132), respectively, in their lyophilized form has been cautiously mixed at room temperature and under controlled sterile conditions with 50.0 g Dimethicone (Dow Corning DC 200 fluid 100 CST & Dow Corning DC EL80-40 ID).

Homogenisation of the oil phase was performed mechanically for 30 s at ca 2500 rpm at room temperature to afford a suspension having a viscosity of ca 100 mPa.s (measured at 20 rpm) and having an initial theoretical concentration of 10¹⁰ CFU/g.

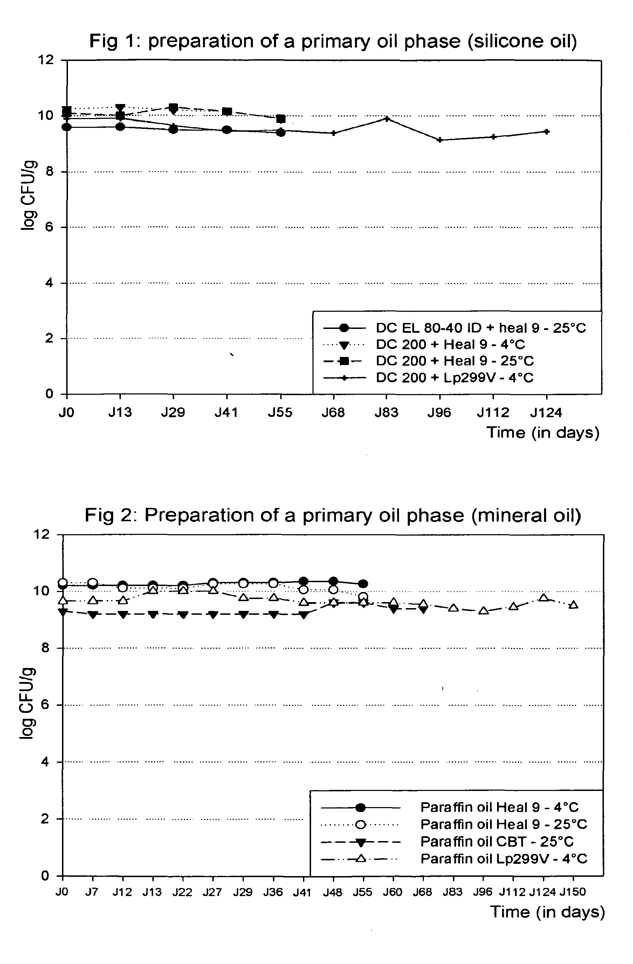

The suspension was then stored at 4° C and 25° C, respectively, over a period of ca 2 to 5 months and samples of same were taken on a weekly basis for analysis**. The results so achieved (see Fig. 1) have shown that, after a short period of decrease of the initial concentration from log 10 to about log 9 CFU/g.

** 1 g of suspension is taken under sterile conditions from the stored material and poured into 9 ml of a suitable solvent (polysorbate / NaCl / sterilized water) and eventually adjusted to 10⁻⁸ by means of sterile physiological water. The 10⁻⁶ to 10⁻⁸ dilutions were then sown by means of MRS culture medium at 45° C and eventually cultured in anaerobic conditions at 37° C over a period of 48 hours for final count.

### Example 2: Preparation of a primary oil phase (mineral oil)

A portion of 0.60 g of either L. plantarum 299v (DSM 9843) or L. plantarum Heal 9 (DSM 15132) or L. plantarum LP(1) (KCTC 3928) in its lyophilized form has been cautiously mixed at room temperature and under controlled sterile conditions with approx 49.95 g of paraffin oil (liquid Vaseline®).

Homogenisation of the oil phase was performed mechanically for 30 s at ca 800 rpm at room temperature to afford a suspension having a viscosity of ca 36 mPa.s (at 20 rpm) and having an initial theoretical concentration of 10¹⁰ CFU/g.

The suspension so afforded and that prepared according top Example 2 ** were then stored at 4° C and 25° C, respectively, over a period of ca 2 to 4 months and samples of same were taken there from on a weekly basis for analysis**. The results so achieved (see Fig. 2) have shown that the concentration of the viable lactobacillus bacteria remain quite stable over the time, i.e. remaining in the range of 10 to log 9.0 CFU/g depending on the tested strain.

### Example 3: Preparation of an alternative primary oil phase (silicone wax)

The same amounts as provided in Example 1 have been used for preparing a primary silicone oil phases using L. plantarum 299v (DSM 9843) and Bis-PEG-18 methyl ether dimethylsilane (Dow Corning 2501 Cosmetic wax).

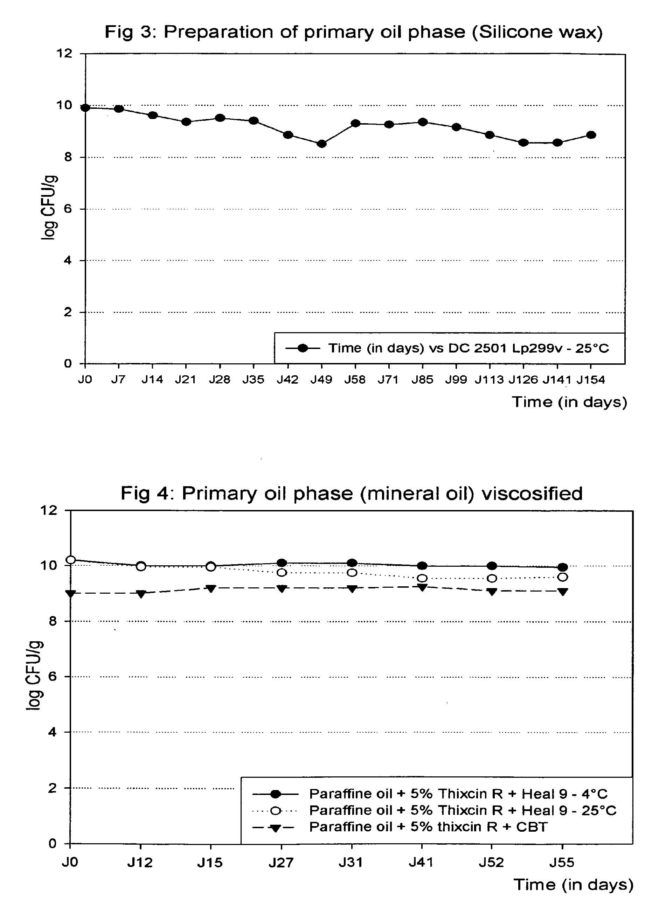

The results so achieved are disclosed in Fig. 3.

### Example 4: Preparation of a viscosified mineral oil primary phase

50 g of a mixture comprising paraffin oil (liquid Vaseline®), 5 % Thixcin has been prepared and the homogenised as follows: mixing for ca 20 min at 60° C under ca 1200 rpm, cooling at 35° C over 10 min under stirring (1500 rpm) and final cooling at room temperature still under stirring.

0.60 g of L. plantarum LP(1) (KCTC 3928) or L. plantarum Heal 9 (DSM 15132) in its lyophilized form have been added to the oil mixture as mentioned here above to afford a suspension having a viscosity of ca 7500 mPa.s (measured at 20rpm) an initial theoretical concentration of 10¹⁰ CFU/g.

One observes that the concentration of viable lactobacillus bacteria remained remarkably stable at this concentration level after 2 months (see Fig. 4).

### Example 5: Preparation of an alternative primary oil phase (silicone blend)

The same amounts as provided in Example 1 have been used for preparing a silicone oil phase using P. acidilactici of Lallemand Rosell in the following blend (weight percent):

| | | | |
|---|---|---|---|
| - | phenyl trimethicone | (DC 556 Cosmetic grade fluid) | 5 % |
| - | cyclomethicone | (Dow Corning DC 345 fluid) | 20 % |
| - | cydomethicone/dimethyconol | (DC 1401 fluid) | 75 % |

The results so achieved are disclosed in Fig. 5.

### Example 6: Preparation of a cosmetic composition (silicone oil/water emulsion)

| **Phase** | **Commercial denomination (or INCI Name)** | **Amounts (g)** |
|---|---|---|
| | | |
| A | Sterilized water | 62.23 |
| | DC* HMW 2220 non ionic emulsion | 4.95 |
| | | |
| | DC* 1401 fluid | 15.10 |
| | DC* 345 fluid | 6.97 |
| B | DC* 556 cosmetic grade fluid | 0.99 |
| | DC* 9701 cosmetic powder | 1.00 |
| | Cremophor CO 40 / PEG-40 hydrogenated castor oil | 4.97 |
| | Nipanox BHT | 0.10 |
| | Gelinov / Sodium polyacrylate & C18-C21 alkane.... | 2.52 |

| | | |
|---|---|---|
| DC* = Dow Corning | | |

1.205 g of L. plantarum 299v (DSM 9843) have been added to phase B (oily phase) and the components thereof mixed at room temperature and eventually homogenized manually. The homogenized phase was then poured by portions into phase A (water phase) under stirring (600 - 800 rpm) over ca 15 min and the above emulsion.

The composition was then stored at 25° C over a period of 5 months and samples of same were taken there from on a weekly basis for analysis**. The results so achieved (see Fig. 6) have shown that, after a short period of decrease of the initial concentration from log 10 to about log 8 CFU/g, the concentration of viable lactobacillus bacteria remained remarkably stable at this concentration level over the long term.

## Claims

1. A device for the extemporaneous preparation of a cosmetic or pharmaceutical composition useful for topical application, said composition consisting of a primary oily phase and a complementary aqueous phase wherein the primary oily phase comprises a suspension of an effective amount of at least one viable probiotic bacteria strain in a non-polar mineral or silicone oil and wherein the at least one probiotic bacteria strain is a lactobacillus strain, which consists of a container comprising two separate compartments each having its own outlet acting as dispensing or dosing or deposition system, wherein the first compartment comprises a primary anhydrous oily phase as defined here above and wherein the second compartment comprises the complementary aqueous phase as defined here above.

2. The device according to claim 1 wherein the primary oily phase is devoid of any exogenous preservative agent.

3. The device according to any of claims 1 to 2 wherein the primary oily phase comprises a sufficient amount of a viscosifying or suspension stabilizing agent.

4. The device according to any of claims 1 to 3 wherein the primary oily phase comprises from 10⁷ to 10¹¹, preferably from 10⁸ to 10¹⁰ colony forming units (CFU) per g of the at least one probiotic lactobacillus strain.

5. The device according to any of claims 1 to 4 wherein the at least one probiotic lactobacillus strain is selected from the group consisting of L. plantarum, L. jensenii, L. gasseri, L. helveticus, L. fermentum, L. rhamnosus, L. casei, L. reuteri or L. acidophilus.

6. The device according to any of claims 1 to 5 wherein the at least one probiotic lactobacillus strain is selected from Lactobacillus plantarum DSM 15312; Lactobacillus plantarum DSM 15313; Lactobacillus plantarum DSM 9843; Lactobacillus plantarum LP(1) KCTC 3928; Lactobacillus jensenii CNCM I-3217; Lactobacillus gasseri CNCM I-3218; Lactobacillus jensenii CNCM I-3219; Lactobacuillus helveticus CNCM I-3360.

7. The device according to any of claims 1 to 6 wherein the complementary aqueous phase is a cosmetic or pharmaceutical solution or emulsion comprising at least one of emulsifiers, stabilizers, jellifying agents, antioxidants, foaming agents or preservatives.

8. The device according to any of the claims to 1 to 7 for use in treating or preventing skin or mucosal infectious disorders or troubles in humans, preferably for treating or preventing urinary tract infections (UTI) and lower vaginal tract infections in females; or for balancing or restoring skin or mucosal indigenous micro flora in humans, preferably for balancing or restoring the local urinary tract and lower vaginal tract bacterial flora in females, wherein extemporaneous preparation comprises the simultaneous deposition of the primary oily phase and of the complementary aqueous phase respectively, and subsequent mixing of same during the application.

## Patentansprüche

1. Vorrichtung zur Ad-hoc-Herstellung einer kosmetischen oder pharmazeutischen Zusammensetzung zum Gebrauch für topische Applikation, wobei die besagte Zusammensetzung aus einer primären Ölphase und einer komplementären wässerigen Phase besteht, wobei die primäre Ölphase eine Suspension einer effektiven Menge von wenigstens einem lebensfähigem probiotischen bakteriellen Stamm in einem unpolaren Mineral- oder Silikonöl umfasst und wobei jener wenigstens ein probiotischer bakterieller Stamm ein Stamm eines Milchsäurebakteriums ist, wobei die Vorrichtung aus einem Behältnis besteht das zwei separate Kompartimente umfasst von denen jedes seine eigenen Auslass hat, der als Abgabe-, Dosierungs oder Ablagesystem wirkt, wobei das erste Kompartiment eine wie oben definierte, primäre, anhydrische Ölphase umfasst und wobei das zweite Kompartiment die wie oben definierte komplementäre Wasserphase umfasst.

2. Die Vorrichtung gemäss Anspruch 1, wobei die primäre Ölphase frei von jedwedem exogenen Konservierungsmittel ist.

3. Die Vorrichtung nach einem der Ansprüche 1 bis 2, wobei die primäre Ölphase eine genügende Menge eines viskosierenden oder suspendierenden Mittels umfasst.

4. Die Vorrichtung nach einem der Ansprüche 1 bis 3, wobei die primäre Ölphase von 10⁷ bis 10¹¹, vorzugsweise von 10⁸ bis 10¹⁰ koloniebildende Einheiten (CFU) des wenigstens einen Milchsäurebakteriums pro Gramm umfasst.

5. Die Vorrichtung nach einem der Ansprüche 1 bis 4, worin der wenigstens eine probiotische Milchsäurebakteriumstamm aus der folgenden Gruppe ausgewählt ist: L. plantarum, L. jensenii, L. gasseri, L. helveticus, L. fermentum, L. rhamnosus, L. casei, L. reuteri, oder L. acidophilus.

6. Die Vorrichtung nach einem der Ansprüche 1 bis 5 wobei der wenigstens eine Milchsäurebakteriumstamm ausgewählt ist von: Lactobacuillus plantarum DSM 15312; Lactobacuillus plantarum DSM 15313; Lactobacuillus plantarum DSM 9843; Lactobacuillus plantarum LP(1) KCTC 3928; Lactobacuillus jensenii CNCM-3217; Lactobacuillus gasseri CNCM I-3218; Lactobacuillus jensenii CNCM-I-3219; Lactobacuillus helveticus CNCM I-3360.

7. Die Vorrichtung nach einem der Ansprüche 1 bis 6, wobei die komplementäre, wässerige Phase eine kosmetische oder pharmazeutische Lösung oder Emulsion ist, die wenigstens einen von Emulgatoren, Stabilisatoren, Geliermittel, Antioxidantien, Schaumbildner oder Konservierungsmittel umfasst.

8. Die Vorrichtung nach einem der Ansprüche 1 bis 7 zur Verwendung in der Behandlung oder Prophylaxe von Haut- oder Mukoseentzündungsstörungen oder - Problemen bei Menschen, vorzugsweise zur Behandlung oder Prophylaxe von Entzündungen der Harnwege (UTI) und des Scheidenvorhofes bei Frauen; oder zum Ausgleich oder zur Wiederherstellung der indigenen Haut- oder Mukosamikroflora in Menschen, vorzugsweise zum Ausgleich oder zur Wiederherstellung der bakteriellen Flora des lokalen Harnweges oder des Scheidenvorhofes bei Frauen, worin die Ad-hoc-Herstellung das simultane Ablegen der primären Ölphase und der komplementären wässerigen Phase, respektive, umfasst, und das darauffolgende Mischen derselben während der Applikation.

## Revendications

1. Un dispositif pour la préparation extemporanée d'une composition cosmétique ou pharmaceutique pour application topique, ladite composition consistant en une phase huile primaire et une phase aqueuse complémentaire dans laquelle la phase huile primaire comprend une quantité efficace d'une suspension d'au moins une souche bactérienne probiotique viable dans une huile minérale ou de silicone non polaire et dans laquelle la au moins une souche bactérienne probiotique est une souche de lactobacille, qui consiste en un récipient comprenant deux compartiments séparés comportant chacun leur propre sortie agissant comme système de distribution ou de dosage ou de dépôt, dans lequel le premier compartiment comprend une phase huile primaire telle que définie ci-dessus et dans lequel le second compartiment comprend la phase aqueuse complémentaire telle que défmie ci-dessus.

2. Le dispositif selon la revendication 1 dans lequel la phase huile primaire est exempte de tout agent conservateur exogène.

3. Le dispositif selon l'une des revendications 1 à 2 dans lequel la phase huile primaire comprend une quantité suffisante d'un agent de viscosité, de suspension ou de stabilisation.

4. Le dispositif selon l'une des revendications 1 à 3 dans lequel la phase huile primaire comprend de 10⁷ à 10¹⁰, de préférence de 10⁸ à 10¹⁰ unités formatrices de colonies (UFC) par g de la au moins une souche bactérienne probiotique.

5. Le dispositif selon l'une des revendications 1 à 4 dans lequel la au moins une souche bactérienne probiotique est choisie à partir du groupe consistant en L. plantarum, L. jensenii, L. gasseri, L. helveticus, L. fermentum, L. rhamnosus, L. casei, L. reuteri ou L. acidophilus.

6. Le dispositif selon l'une des revendications 1 à 5 dans lequel la au moins une souche bactérienne probiotique est choisie à partir de Lactobacillus plantarum DSM 15312, Lactobacillus plantarum LP(1) KCTC 3028, Lactobacillsu jensenii CNCM I-3217, Lactobacillus gasseri CNCM I-3218, Lactobacillus jensenii CNCM I-3219, Lactobacillus helveticus CNCM I-3360.

7. Le dispositif selon l'une des revendications 1 à 6 dans lequel la phase aqueuse complémentaire est une solution ou émulsion cosmétique ou pharmaceutique comprenant au moins un des agents émulsifiants, stabilisants, gélifiants, antioxydants, agents moussants ou conservateurs.

8. Le dispositif selon l'une des revendications 1 à 7 pour usage dans le traitement ou la prévention de désordres ou de troubles infectieux de la peau ou des muqueuses chez l'être humain, de préférence dans le traitement ou la prévention d'infections des voies urinaires (UTI) ou des voies vaginales basses chez la femme, ou pour équilibrer ou restaurer la microflore bactérienne indigène de la peau ou des muqueuses chez l'être humain, de préférence pour équilibrer ou restaurer la flore bactérienne locale des voies urinaires et des voies vaginales basses chez la femme, dans lequel la préparation extemporanée comprend le dépôt simultané de la phase huile primaire et de la phase aqueuse complémentaire respectivement et le mélange subséquent de celles-ci au cours de l'application.
